Europäisches Patentamt

European Patent Office

Office européen des brevets

(11) Veröffentlichungsnummer: **0 273 180 B1**

(12) **EUROPÄISCHE PATENTSCHRIFT**

(45) Veröffentlichungstag der Patentschrift: **12.06.91**

(51) Int. Cl.5: **G10K 11/00, G01S 15/89, A61B 8/12**

(21) Anmeldenummer: **87117100.5**

(22) Anmeldetag: **19.11.87**

(54) **Intrakavitäre Ultraschall-Abtasteinrichtung.**

(30) Priorität: **05.12.86 DE 3641280**

(43) Veröffentlichungstag der Anmeldung:
**06.07.88 Patentblatt 88/27**

(45) Bekanntmachung des Hinweises auf die
Patenterteilung:
**12.06.91 Patentblatt 91/24**

(84) Benannte Vertragsstaaten:
**AT CH DE FR GB LI NL**

(56) Entgegenhaltungen:
**DE-A- 3 537 904**
**DE-A- 3 618 082**
**DE-A- 3 618 906**
**GB-A- 1 578 405**

**IEEE 1974 ULTRASONICS SYMPOSIUM PRO-
CEEDINGS, Katalog Nr. 74 CHO 896-1SU;
1974, Seiten 744-748, New York, US; F.E. BAR-
BER et al.: "Duplex scanner II: for simultaneous imaging of artery tissues and flow"**

**ULTRASOUND IN MEDICINE & BIOLOGY,
Band 5, 1979, Seiten 129-138, Pergamon
Press LTD, Oxford, GB; W.B. TAYLOR et al.:**

**"A high-resolution transrectal ultrasonographic system"**

(73) Patentinhaber: **Siemens Aktiengesellschaft
Wittelsbacherplatz 2
W-8000 München 2(DE)**

(72) Erfinder: **Hetz, Walter, Dipl.-Ing.
Adam-Kraft-Strasse 17
W-8520 Erlangen(DE)**

**Beschreibung**

Die Erfindung bezieht sich auf eine intrakavitäre Ultraschall-Abtasteinrichtung mit einem Untersuchungskopf, in dessen Innenraum ein Ultraschall-Wandler untergebracht ist, der um eine Achse drehbar und dabei mittels einer Antriebsachse antreibbar ist, wobei die genannte Achse im wesentlichen senkrecht zum distalen Ende der Antriebsachse ausgerichtet ist und wobei zum Drehen des Ultraschall-Wandlers um die Achse ein erstes Zahnrad vorgesehen ist, das mit einem zweiten, am distalen Ende der Antriebsachse befestigten Zahnrad im Eingriff steht.

Ultraschall-Abtasteinrichtungen der genannten Art (Ultrasound in Med. & Biol., Vol. 5, pp. 129 - 138, "A High-Resolution Transrectal Ultrasonographie System") sind auf dem Markt erhältlich. Sie enthalten in der Regel einen einzigen mechanisch bewegten Ultraschall-Wandler, der entweder hin- und hergeschwenkt oder aber rotiert wird. Mit solchen Abtasteinrichtungen werden Körperhöhlen, z.B. das Rektum oder die Vagina, untersucht. Daraus ergibt sich die Forderung, daß der Untersuchungskopf bei hohem Leistungsvermögen möglichst klein aufgebaut sein soll.

Nach FIG 2 und 3 der DE-OS 36 18 082 ist bei einer Ultraschall-Meßkopfanordnung für die medizinische Diagnostik ein Ultraschall-Wandler am Ende der Meßkopfanordnung auf einer Achse drehbar gelagert, die senkrecht zu einer Antriebsachse ausgerichtet ist. Der Antrieb des Wandlers erfolgt über ein Kegelradgetriebe. Es ist dort jedoch kein zweiter Ultraschall-Wandler vorhanden, mit dem eine Beobachtung in einer zweiten Schnittebene möglich ist. Außerdem eignet sich die gezeigte Meßkopfanordnung wegen der großen Außenabmessungen nicht für intracavitäre Untersuchungen.

Es ist auch bereits eine intrakavitäre Ultraschall-Abtasteinrichtung aus der Europäischen Patentveröffentlichung 0,139,574 bekannt geworden, bei der mit zwei Ultraschall-Wandleranordnungen gearbeitet wird. Die Untersuchungs flächen oder Schnittebenen stehen bei der bekannten Einrichtung senkrecht aufeinander. Bei der bekannten Einrichtung ist der eine Ultraschall-Wandler mechanisch bewegt, während mit dem anderen Ultraschall-Wandler, einem linearen Array, eine elektronische Abtastung durchgeführt wird. Die Betrachtung zweier aufeinander senkrecht stehender Schnittebenen ermöglicht dem Beobachter eine präzisere Diagnose als eine Untersuchung in lediglich einer einzigen Abtastebene. Größe und Lage interessierender Objekte lassen sich auf diese Weise leichter feststellen.

Es war bereits erwähnt worden, daß ein Untersuchungskopf der genannten Art eine möglichst geringe Baugröße aufweisen sollte.

Aufgabe der vorliegenden Erfindung ist es, eine Ultraschall-Abtasteinrichtung der eingangs genannten Art anzugeben, bei der der Untersuchungskopf so ausgebildet ist, daß eine genaue Beobachtung und Untersuchung des interessierenden Gebietes in zwei Abtastebenen möglich ist, ohne daß die Lage der Ultraschall-Abtasteinrichtung verändert wird. Dabei soll der Untersuchungskopf eine gedrungene Bauform besitzen. Er soll also - in longitudinaler Richtung der Antriebsachse gesehen - verhältnismäßig kurz sein. Seine eine Abtastebene soll dabei in dieser longitudinalen Richtung (oder parallel dazu versetzt) liegen, die andere schräg oder ungefähr senkrecht dazu.

Diese Aufgabe wird erfindungsgemäß dadurch gelöst, daß ein mit der Antriebsachse verbundenes Halteteil vorgesehen ist, an dem ein weiterer Ultraschall-Wandler befestigt ist.

Die Abstrahlrichtung des weiteren Ultraschall-Wandlers kann dabei schräg zur Radialebene der Antriebsachse ausgerichtet sein. Auf diese Weise erhält man einen mechanischen Ultraschall-Scanner für Körperhöhlen mit der Möglichkeit, in zwei Untersuchungsflächen abzutasten, die entweder senkrecht oder - wie erwähnt - beinahe senkrecht aufeinander stehen. Bei dieser Konstruktion ist auch erreicht, daß die Drehpunkte der Sektoren, die man auf den beiden Bildschirmen sieht, recht nahe aneinander liegen. Gleichzeitig ist dafür gesorgt, daß man mit einem einzigen Antrieb, nämlich der Antriebswelle, für beide Ultraschall-Wandler auskommt.

Dadurch, daß hier zwei im wesentlichen senkrecht aufeinander stehende Zahnräder verwendet werden, wird eine gedrungene Bauform des Untersuchungskopfes erreicht. Mit dem Begriff "Achse" ist dabei eine raumfeste Orientierungslinie gemeint. In der praktischen Realisierung handelt es sich z.B. um eine drehbare Welle oder um eine raumfest angeordnete Lagerachse.

Prinzipiell können hierbei schrägverzahnte Zahnräder verwendet werden. Nach einer bevorzugten Ausgestaltung jedoch sind das erste und zweite Zahnrad ein erstes bzw. zweites Kegelrad, und das zweite Kegelrad weist eine distal gelegene Mulde auf, in die der Ultraschall-Wandler beim Drehen um die Achse eintaucht. Durch Verwendung der genannten Mulde wird also Platz in longitudinaler Richtung gespart.

Eine weitere vorteilhafte Ausgestaltung zeichnet sich dadurch aus, daß der erste Ultraschall-Wandler fest mit der Antriebsachse gekoppelt ist und daß der zweite Ultraschall-Wandler über eine lösbare Kupplung mit der Antriebsachse gekoppelt ist. In einer Weiterbildung ist der zweite Ultraschall-Wandler, wenn er mit der Antriebsachse gekoppelt ist, zur Erzeugung eines B-Bildes, und wenn er von der Antriebsachse abgekoppelt ist, zur Durchführung einer Doppler-Flußmessung vorgesehen. Von Vorteil ist, daß die Ausrichtung des Untersuchungskopfes auf das zu untersuchende Gefäß in zwei Untersuchungsebenen genau beobachtbar ist. Während der darauf folgenden Doppler-Flußmessung kann über die Sektorabtastung des ersten Ultraschall-Wandlers gleichzeitig eine Lageveränderung des Untersuchungskopfes oder des Gefäßes im Körper sofort erkannt und dann korrigiert werden.

Eine weitere vorteilhafte Ausführungsform der intrakavitären Ultraschall-Abtasteinrichtung ergibt sich, wenn eine Biopsienadelführung, die ein Führungsrohr und eine Klemmvorrichtung umfaßt, mit dem Untersuchungskopf lösbar verbunden ist, wenn das Führungsrohr seitlich versetzt und in Richtung der Antriebsachse angeordnet ist und wenn die Auslaßöffnung des Führungsrohrs schräg zur Antriebsachse ausgerichtet ist. Hier ist die Kombination der Ultraschall-Abtasteinrichtung mit der Biopsienadelführung besonders vorteilhaft, weil bei der Durchführung der Biopsie die Biopsienadel über die beiden senkrecht oder beinahe senkrecht aufeinander stehenden Untersuchungsflächen beobachtbar ist. Es können somit Hindernisse im Zuführweg der Biopsienadel, die in der einen Untersuchungsfläche nicht sichtbar sind, in der anderen Untersuchungsfläche erkannt werden. Die Ausrichtung der Auslaßöffnung des Führungsrohres schräg zur Antriebsachse erlaubt es, Gewebe- oder Flüssigkeitsproben über die Wandung der Körperhöhle aus dem Innern des Körpers zu entnehmen.

Weitere vorteilhafte Ausgestaltungen der Erfindung sind in den Unteransprüchen niedergelegt. Ausführungsbeispiele werden im folgenden anhand von FIG näher erläutert. Es zeigen:

FIG 1 die Seitenansicht einer intrakavitären Ultraschall-Abtasteinrichtung nach der Erfindung,

FIG 2 einen Schnitt durch die Ultraschall-Abtasteinrichtung nach FIG 1,

FIG 3 einen zum Schnitt in FIG 2 senkrechten Schnitt durch eine Ultraschall-Abtasteinrichtung, die zur Doppler-Flußmessung vorgesehen ist und bei der ein Ultraschallwandler über eine Kupplung mit der Antriebsachse gekoppelt ist,

FIG 4 eine Monitor-Abbildung der Untersuchungsfläche, die von der Ultraschall-Abtasteinrichtung nach FIG 3 erzeugt wird und die in FIG 3 mit A-A bezeichnet ist,

FIG 5 eine Draufsicht, teilweise im Schnitt dargestellt, auf einem Untersuchungskopf einer Ultraschall-Abtasteinrichtung mit einer Biopsienadelführung, und

FIG 6 eine Vorderansicht der Biopsienadelführung nach FIG 5.

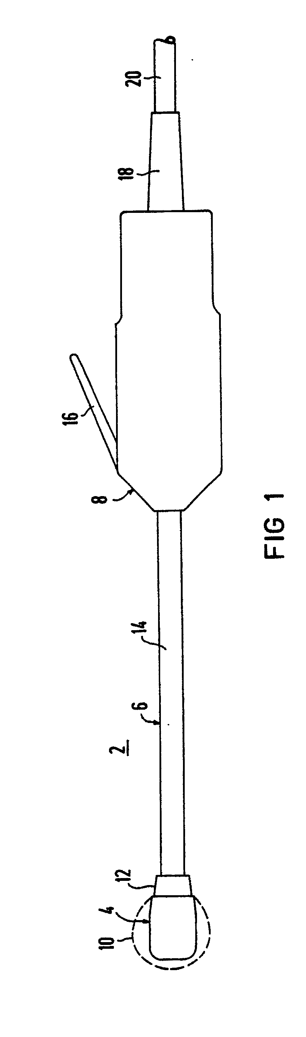

Nach FIG 1 umfaßt eine Ultraschall-Abtasteinrichtung 2 für Körperhöhlungen als wesentliche Bestandteile einen Untersuchungskopf 4, eine geradlinige Führung 6 und ein Handstück oder einen Gehäusekopf 8. Die Ultraschall-Abtasteinrichtung 2 ist - wie im folgenden näher erläutert wird - so ausgebildet, daß nach Einführung des Untersuchungskopfes 4 in die Körperhöhlung in zwei Untersuchungsflächen (die im wesentlichen senkrecht aufeinander stehen) untersucht werden kann, ohne daß die Abtasteinrichtung 2 bei Betrachtung eines Objekts in longitudinaler Richtung der Führung 6 beim Übergang (z.B. Umschalten) von der ersten Untersuchungsfläche in die zweite Untersuchungsfläche bewegt zu werden braucht. Die beiden Untersuchungsebenen können nacheinander oder auch gleichzeitig auf einem/zwei Bildschirm(en) dargestellt werden (z.B. als konventionelle Sektorbilder). Der Untersuchungskopf 4 ist in longitudinaler Richtung besonders kurz ausgebildet. Das hat zur Folge, daß die Drehpunkte der beiden auf dem Bildschirm oder den Bildschirmen dargestellten Sektoren sehr eng beieinander liegen.

Aus FIG 1 ist ersichtlich, daß der Untersuchungskopf 4 von einer Umhüllung 10 aus einem elastischen Material, z.B. Gummi, umgeben ist. Diese Umhüllung 10 kann in bekannter Weise aufgeblasen werden, um ein gutes Anliegen an der Höhlungswand und damit eine gute Ultraschall-Ankopplung zu erreichen. Der aufgeblasene Zustand der Umhüllung 10 ist gestrichelt eingezeichnet. Die Umhüllung 10 ist mittels einer Klemmutter 12 am Untersuchungskopf 4 festgehalten. Die Führung 6 ist starr ausgebildet und verbindet den Untersuchungskopf 4 mit dem Gehäusekopf 8. Sie ist außen mit einer Kunststoff-Umhüllung 14 versehen.

Der Gehäusekopf 8 enthält in bekannter Weise einen Elektromotor mit einem Positionsgeber und dient bei der Untersuchung als Manipulator. Vorliegend enthält er noch eine (nicht gezeigte) Pumpe zum Aufpumpen der Umhüllung 10 über die Führung 6. Von dieser Pumpe ist lediglich der Pumpenbetätigungshebel 16 dargestellt. Stattdessen kann es sich bei diesem Hebel 16 auch um einen Hebel zur Betätigung eines Ventils zwecks Aufblasens der Umhüllung 10 oder zwecks Ablassens des Druckes darin handeln. Elektrische Verbindungsleitungen führen vom Gehäusekopf 8 durch die Führung 6 zum Untersuchungskopf 4; weitere Verbindungsleitungen sind zur Leitung von Signalen vom Untersuchungskopf 4 durch die Führung 6 zum Gehäusekopf 8 vorgesehen. All diese Leitungen sind nach Durchgang durch eine Knickschutztülle 18 in Form eines elektrischen Anschlußkabels 20 mit einem (an sich bekannten) Ultraschall-B-Bild-Gerät verbunden. Dieses dient in herkömmlicher Weise zur Erzeugung von Sendimpulsen, zur Signalauswertung und Bilddarstellung. Das B-Bildgerät ist zwischen zwei im folgenden erwähnten Ultraschall-Schnittbildern umschaltbar. Es könnten stattdessen auch zwei Schnittbilder in Echtzeit dargestellt werden.

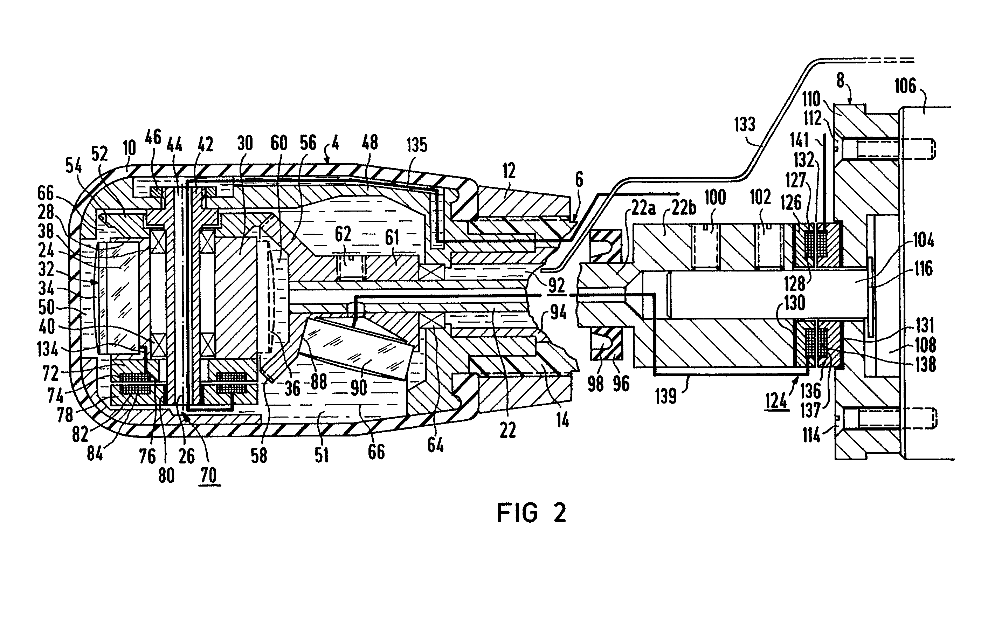

In FIG 2 sind Einzelheiten des Untersuchungskopfes 4, der Führung 6 und des Gehäusekopfes 8 im Schnitt dargestellt. Die Verkleidung des Gehäusekopfes 8 ist dabei nicht gezeichnet.

Nach FIG 2 dient eine vom Gehäusekopf 8 zum Untersuchungskopf 4 verlaufende Antriebsachse 22 zum Drehen eines ersten Ultraschall-Wandlers 24 um eine Achse 26. Die Achse 26 ist senkrecht zum distalen Endabschnitt der Antriebsachse 22 ausgerichtet. Die Antriebsachse 22 ist als Hohlachse ausgeführt. Sie liegt zentral in der Führung 6 und besteht bevorzugt aus einem Metall.

Der erste Ultraschall-Wandler 24 ist zylindrisch ausgebildet. Er sitzt in einer Senkung 28, die in einem zylindrischen Körper oder einer Trommel 30 untergebracht ist. Diese Trommel 30 kann sich um die Achse 26 drehen. Die Abstrahlfläche 32 des ersten Ultraschall-Wandlers 24 ist mit einer Linse 34 belegt. Diese kann beispielsweise aus Silikon-Kautschuk bestehen. Anstelle eines einzigen Ultraschall-Wandlers 24 können auch mehrere solche Wandler am Umfang der Trommel 30 versetzt angeordnet sein. In der gezeichneten Stellung weist die Abstrahlfläche 32 des Ultraschall-Wandlers 24 nach links. Beim Drehen um die Achse 26 wird somit eine Untersuchungsebene abgetastet, die senkrecht auf der Papierebene steht und im gezeigten Ausführungsbeispiel speziell mit der Längsachse der Antriebsachse 22 zusammenfällt. Die um 180° gedrehte Stellung der Abstrahlfläche 32 ist gestrichelt eingezeichnet und mit 36 bezeichnet. Sie entspricht der maximalen Eintauchposition des ersten Ultraschall-Wandlers 24 in das Innere des Untersuchungskopfes 4 beim Drehen um die Achse 26.

Die als Schallkopfträger dienende Trommel 30 ist mittels zweier Kugellager 38, 40 auf einer Lagerachse 42 gelagert. Diese Lagerachse 42 ist als Hohlachse ausgebildet, um die Durchführung von elektrischen Leitungen zu ermöglichen. Ihre Symmetrieachse ist die Achse 26, die zentral durch den Kanal 44 verläuft. Die Lagerachse 42 ist raumfest im Inneren des Untersuchungskopfes 4 angeordnet. Dazu ist sie an einem Ende z.B. mittels einer Mutter 46 an einem Gehäuse 48 verschraubt. Dieses Gehäuse 48 kann insbesondere aus Kunststoff, vorzugsweise aber aus Metall, bestehen. Es besitzt ein erstes Ultraschall-Fenster 50, das an der distalen Stirnwand angeordnet und für den ersten Ultraschall-Wandler 24 vorgesehen ist, sowie ein zweites (nicht gezeichnetes) Ultraschall-Fenster 51, das in radialer Richtung liegt. Das Gehäuse 48 ist an einem Tragrohr 94 befestigt.

An der einen Stirnseite der Trommel 30 ist ein erstes Zahnrad 52 nahe der Gehäusewand befestigt. Trommel 30 und Zahnrad 52 drehen sich also gemeinsam um die Achse 26. Das erste Zahnrad 52 ist insbesondere als Kegelrad ausgebildet. Die Verzahnung 54 liegt hier unter einem Winkel von 45° zur Achse 26. Dieses erste Zahnrad 52 steht an der Verzahnung 54 mit einem zweiten Zahnrad 56 im Eingriff, das am distalen Ende der Antriebsachse 22 befestigt ist. Dieses zweite Zahnrad 56 ist ebenfalls als Kegelrad ausgebildet. Es besitzt zwischen seiner Verzahnung 58 eine Ausnehmung oder Mulde 60, durch die der erste Ultraschall-Wandler 24 hindurchgedreht wird. Infolge der Mulde 60 kann eine verhältnismäßig kleine Baugröße in longitudinaler Richtung der Antriebsachse 22 erreicht werden. Das zweite Zahnrad 56 besitzt am proximalen Ende ein Halteteil 61, das mittels einer Madenschraube 62 auf der Antriebsachse 22 befestigt ist. Eines der beiden miteinander kämmenden Zahnräder 52 und 56 besteht insbesondere aus einem Metall, das andere aus einem Kunststoff, um die Geräuschentwicklung gering zu halten. Das zweite Zahnrad 56 liegt noch innerhalb des Gehäuses 48. An seinem Ende befindet sich ein weiteres Kugellager 64, das am Gehäuse 48 befestigt ist und die Antriebsachse 22 in ihrem Endbereich zentral führt. Sämtliche Kugellager 38, 40 und 64 sind durchlässig für eine Koppelflüssigkeit 66, die zu einer guten Ultraschall-Übertragung dient und mit der der Innenraum des Untersuchungskopfes 4 und der Führung 6 gefüllt ist.

Es ist erforderlich, den ersten Ultraschall-Wandler 24 mit elektrischen Impulsen zu versorgen und elektrische Signale von diesem abzuleiten. Zu diesem Zweck ist ein Drehtransformator 70 vorgesehen. Dieser Drehtransformator 70 besteht aus einem drehenden und einem nicht-drehenden Teil. Das drehende Teil umfaßt ein ringförmiges Tragteil 72, das an der zweiten Stirnseite der Trommel 30 befestigt ist. In einer Ringnut 74 liegt eine Übertragerspule 76. Durch einen geringen Luftspalt getrennt hiervon befindet sich das feststehende Teil des Drehtransformators 70. Dieses umfaßt ein ringförmiges Tragteil 78, das mittels eines Klebers 80 am Ende der feststehenden Lagerachse 42 befestigt ist. Es enthält entsprechend eine Ringnut 82, in der eine weitere Spule 84 untergebracht ist. Mit Hilfe des Drehtransformators 70 ist eine nicht-galvanische Signalübertragung möglich.

Im Halteteil 61 des zweiten Zahnrades 56 ist eine Senkung 88 vorgesehen. In dieser Senkung 88 befindet sich ein zweiter zylindrischer Ultraschall-Wandler 90. Es ist bemerkenswert, daß der zweite Ultraschall-Wandler 90 in der Nähe des ersten Ultraschall-Wandlers 24 untergebracht ist. Damit liegen die Ursprünge der beiden zentralen Abstrahlachsen relativ eng beieinander. Auch ist zu betonen, daß dieser zweite Ultraschall-Wandler 90 etwas schräg zur Antriebsachse 22 ausgerichtet ist. Das hat zur Folge, daß der von diesem Ultraschall-Wandler 90 wiederholt ausgesandte Ultraschall-Strahl bei Drehung der Antriebsachse 22 einen Kegelmantel beschreibt, der zentral bezüglich der Antriebsachse 22 liegt. Die Schrägstellung des zweiten Ultraschall-Wandlers 90 ist gewählt, um das interessierende Objekt in den von den Ultraschall-Wandlern 24, 90 gewonnenen Bildern zu erfassen, ohne daß dazu die Lage des Untersuchungskopfes 4 bezüglich des besagten Objekts geändert werden muß. Da die Abtastfläche des zweiten Ultraschall-Wandlers 90 die Abtastebene des ersten Ultraschall-Wandlers 24 schräg schneidet, kann ein Objekt in den zugehörigen Bildschirmbildern gleich gut beobachtet werden (zentrale Lage).

Als nächstes wird noch einmal die Führung 6 betrachtet. Sie umfaßt die im Kugellager 64 geführte Antriebsachse 22 zur Drehung beider Ultraschall-Wandler 24, 90. Diese Antriebsachse 22 ist im Bereich des Gehäusekopfes 8 stufenförmig vergrößert und in der Wandstärke verstärkt. Der verstärkte Antriebsachsenabschnitt ist mit 22a bezeichnet. Die Antriebsachse 22 ist unter Freilassung eines Zwischenraums 92 von einem feststehenden Trägerrohr 94 umgeben. Dieses kann insbesondere aus Metall bestehen. Auf das Trägerrohr 94 ist die flexible Umhüllung 14 geschoben. Auf dem verstärkten Antriebsachsenabschnitt 22a befindet sich ein Dichtungsring 96, der mit einer Ringnut 98 auf der Seite des Untersuchungskopfes 4 versehen ist. Bis in diese Ringnut 98 steht die Ankoppelflüssigkeit 66.

An den Abschnitt 22a schließt ein Endabschnitt 22b der Antriebsachse 22 an. Dieser ist in der Wandstärke weiter verstärkt. Auch der Innendurchmesser ist vergrößert. Der Abschnitt 22b ist mit Hilfe von zwei Madenschrauben 100, 102 auf der Achse 104 eines Elektromotors 106 befestigt. Der Servomotor106 befindet sich im Gehäusekopf 8. An seinem Bund 108 ist ein Flansch 110 mit Hilfe zweier Senkschrauben 112, 114 befestigt. Ein eingelegter Sprengring ist mit 116 bezeichnet.

Auf der Achse 104 befindet sich ein zweiter Drehtransformator 124. Dieser zweite Drehtransformator 124 ist für die Signalvermittlung vom und zum zweiten Ultraschall-Wandler 90 vorgesehen. Das drehbare Teil des Drehtransformators 124 besteht aus einem Tragring 126, in dessen stirnseitiger Ringnut 127 eine erste Übertragerspule 128 eingelassen ist. Der Tragring 126 kann beispielsweise mittels eines Klebers 130 auf der Stirnfläche des Endabschnitts 22b aufgeklebt sein. In einem kurzen Abstand hierzu (Spalt 132) befindet sich das nicht-drehbare Teil des zweiten Drehtransformators 124. Dieses zweite Teil umfaßt entsprechend einen Tragring 136, in dessen Ringnut 137 eine Spule 138 eingelassen ist. Der Tragring 136 ist stirnseitig mittels eines Klebers 131 am Flansch 110 befestigt. In FIG 2 ist auch angedeutet, daß innerhalb des Gehäusekopfes 8 eine hydraulische Leitung 133 angeordnet ist. Sie führt von der erwähnten Handpumpe, deren Pumpenbetätigungshebel 16 in FIG 1 gezeigt ist, zum Zwischenraum 92. Mit Hilfe dieser Pumpe wird über die hydraulische Leitung 133 Ankoppelflüssigkeit 66 in den Zwischenraum 92 und damit in das Gehäuse 48 gepumpt. Die Druckerhöhung hat zur Folge, daß die mit Hilfe der Klemm-Mutter 12 am Gehäuse 48 eingespannte Umhüllung 10 aufgebläht wird und sich somit bei einer Ultraschall-Untersuchung an die Wand der untersuchten Körperhöhlung anschmiegt. Auf diese Weise ist im Bereich der beiden Ultraschall-Fenster 50, 51 ein guter Ultraschall-Übergang möglich.

In der FIG 2 ist auch schematisch der elektrische Leitungsverlauf für Signale zum und vom ersten bzw. zweiten Ultraschall-Wandler 24 bzw. 90 verdeutlicht. Die Leitungen 134, 135 bzw. 139, 141 sind jeweils durch stärkere Linien verdeutlicht. Es ist ersichtlich, daß die elektrischen Leitungen 135 sowohl durch den Kanal 44 in der Lagerachse 42 als auch im Raum zwischen Gehäuse 48 und Umhüllung 10 verlaufen. Sie verlaufen weiterhin radial durch die Gehäusewand und schließlich durch die Kunststoff-Umhüllung 14. Die Leitungen 139 zwischen zweitem Ultraschall-Wandler 90 und zweitem Drehtransformator 124 verlaufen durch die Längsbohrung der Antriebsachse 22 und dann auf dem Außenmantel des verdickten Endstücks 22b. Die Leitungen 135, 141 gelangen schließlich in den Innenraum des Gehäusekopfes 8.

Der Vorteil der in FIG 1 und 2 dargestellten intrakavitären Ultraschall-Abtasteinrichtung 2 wird insbesondere in ihrem einfachen Aufbau und in ihrer raumsparenden, kompakten Konstruktion gesehen. Weiterhin ist von Bedeutung, daß für die mechanische Bewegung der beiden Ultraschall-Wandler 24 und 90, die verschiedene Untersuchungsflächen abtasten, lediglich eine einzige motorisch angetriebene Antriebsachse 22 vorgesehen ist. Um beim Betrieb Störungen auszuschalten, wird man darauf achten, daß die beiden Ultraschall-Wandler 24, 90 wechselweise eingeschaltet sind.

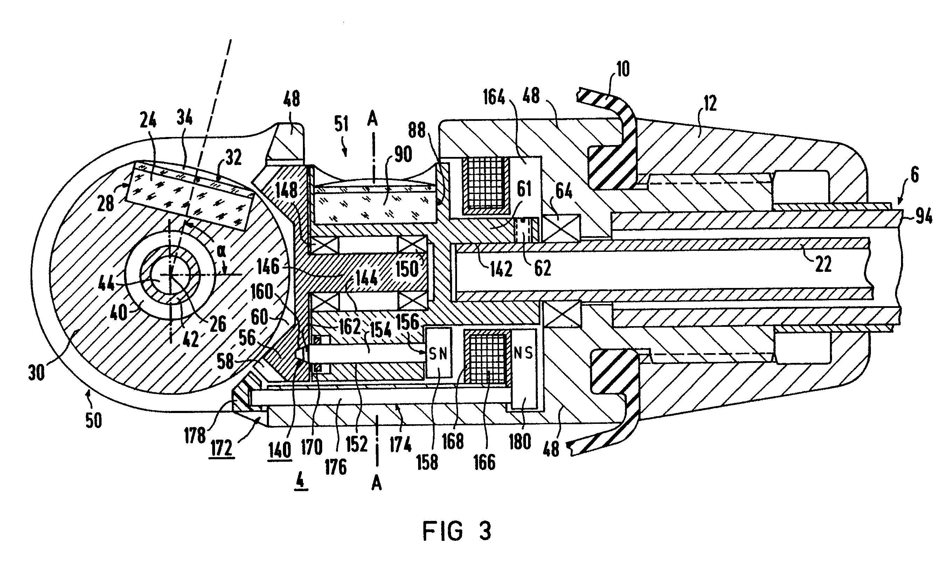

Das Ausführungsbeispiel nach FIG 3 erlaubt sowohl eine Ultraschall-Abtastung in zwei Untersuchungsflächen als auch die Abtastung in nur einer Untersuchungsfläche bei gleichzeitiger Flußmessung nach dem Dopplerprinzip. Der Untersuchungskopf 4 ist ähnlich aufgebaut wie in FIG 2 dargestellt. Für gleiche Teile werden die gleichen Bezugszeichen verwendet. Hier ist der erste Ultraschall-Wandler 90 ebenfalls über das Halteteil 61 fest mit der Antriebsachse 22 gekoppelt. Der zweite Ultraschall-Wandler 24 ist jedoch über eine lösbare Kupplung 140 mit der Antriebsachse 22 gekoppelt.

Zur besseren Übersicht ist die Umhüllung 10 des Untersuchungskopfes 4 in FIG 3 nur teilweise gezeigt. Sie umgibt hier jedoch ebenfalls den gesamten vorderen Teil des Untersuchungskopfes 4.

Das Halteteil 61 ist hier nicht - wie in FIG 2 - mit seiner gesamten Länge über die Antriebsachse 22 gesteckt, sondern weist ein Sackloch 142 auf, in dem sich die Antriebsachse 22 befindet. Auf der dem Sackloch 142 gegenüberliegenden Seite ist ein weiteres Sackloch oder eine zentrale Bohrung 144 in das Halteteil 61 eingebracht. Das zweite Kegelrad 56 ist hier mit einem zentralen Zapfen 146 versehen, der in das Sackloch 144 hineinragt und dort gelagert ist. Die Lagerung erfolgt hier über zwei weitere Kugellager 148, 150.

Der Außendurchmesser des Halteteils 61 vergrößert sich im Bereich des weiteren Sacklochs 144. Dadurch ist im Halteteil 61 Platz geschaffen, daß der erste Ultraschall-Wandler 90 in der Senkung 88 vollständig eingebracht ist. Die Abtastrichtung des Ultraschall-Wandlers 90 ist hier - anders als bei FIG 2 beschrieben - senkrecht zur Antriebsachse 22 ausgerichtet.

Das Kegelrad 56 ist mit dem Halteteil 61 über die im folgenden beschriebene Kupplung 140 lösbar verbunden. Gegenüber der Senkung 88 ist eine Bohrung 152 vorhanden. Diese Bohrung 152 ist seitlich versetzt zu den Sacklöchern 142 und 144 und parallel dazu ausgerichtet.

In der Bohrung 152 befindet sich ein Stift 154. Der Stift 154 ist in der Bohrung 152 verschiebbar. An dem Ende 156 des Stiftes 154, das dem Kegelrad 56 abgewandt ist, ist ein blockförmiger erster Dauermagnet 158 befestigt. Der Nordpol des Dauermagneten 158 ist mit N und der Südpol mit S benannt.

Im Kegelrad 56 ist dem Stift 154 gegenüberliegend eine kegelförmige Aussparung 160 eingebracht. In der FIG 3 greift der Stift 154 mit seinem Ende 162 in die Aussparung 160 ein. Über diesen Stift 154 und das Halteteil 61 ist das zweite Zahnrad 56 mit der Antriebsachse 22 mechanisch verbunden.

Innerhalb des Gehäuses 48 ist im Bereich des Sacklochs 162 ein Freiraum 164 geschaffen. In diesem Freiraum 164 befindet sich konzentrisch zur Antriebsachse 22 eine elektromagnetische Ringspule 166, die auf einen Spulenkörper 168 gewickelt ist. Die Ringspule 166 ist über dem Spulenkörper 168 im Gehäuse 48 befestigt. Der Innendurchmesser des Spulenkörpers 168 ist etwas größer als der Außendurchmesser des Halteteils 61, so daß sich das Halteteil 61 frei drehen kann.

Die Ringspule 166 ist bezüglich des Dauermagneten 158 so angeordnet, daß bei stromdurchflossener Ringspule 166 bei jeder Position des drehbaren Halteteils 61 eine gute magnetische Wechselwirkung mit dem Dauermagneten 158 vorhanden ist, d.h. Dauermagnet 158 und Ringspule 166 sind in geringem räumlichen Abstand zueinander angeordnet.

Wird die Ringspule 166 durch eine erste Stromflußrichtung erregt, ergibt sich eine magnetische Feldverteilung, die den Dauermagneten 158 von der Ringspule 166 wegdrückt; d.h. bei der in der FIG 3 angegebenen Polarisierung des Dauermagneten 158 wird von der Ringspule 166 an der dem Dauermagneten 158 gegenüberliegenden Seite ein magnetischer Nordpol erzeugt. Dabei verbindet die Kupplung mechanisch das zweite Zahnrad 56 über den Stift 154 und das Halteteil 61 mit der Antriebsachse 22. In dieser Stellung des Stiftes 154 ist also der zweite Ultraschall-Wandler 24 mit der Antriebsachse 22 gekoppelt und rotiert bei Drehung der Antriebsachse 22 um die Achse 26. Gleichzeitig rotiert der Ultraschall-Wandler 90 um die Längsachse der Antriebsachse 22. Es können jetzt zwei Untersuchungsebenen, die hier senkrecht aufeinander stehen und sich teilweise kreuzen, auf dem Bildschirm dargestellt werden, wie beim Ausführungsbeispiel nach FIG 2 beschrieben.

Wird die Richtung des Stromes in der Ringspule 166 umgedreht, also die Ringspule 166 mit einem Strom in der zweiten Stromflußrichtung beaufschlagt, ändert sich auch das von der Ringspule 166 erzeugte Magnetfeld. Dem Dauermagneten 158 steht jetzt ein von der Ringspule 166 erzeugter magnetischer Südpol gegenüber. Der Stift 154 wird aus der Aussparung 160 in Richtung zur Ringspule 166 herausgezogen. Ein Anschlag 170, der fest mit dem Stift 154 verbunden ist, erlaubt eine Bewegung des Stiftes 154 in Richtung der Magnetspule 166 nur soweit, bis er gegen den Boden eines erweiterten Teils der Bohrung 152 stößt. Dadurch wird verhindert, daß der Dauermagnet 158 den Spulenkörper 168 berührt.

Eine Bremse 172 verhindert ein Drehen des zweiten Ultraschall-Wandlers 24, wenn er von der Antriebsachse 22 abgekoppelt ist. Dazu ist im Gehäuse 48 parallel zur Antriebsachse 22 eine Bohrung 174 eingebracht. Die Bohrung erstreckt sich im Gehäuse vom Ultraschall-Fenster 50 bis zum Freiraum 164, d.h. die Länge der Bohrung 174 entspricht ungefähr dem Abstand von dem Kegelrad 56 zur Ringspule 166. Am Ultraschall-Fenster 50 ragt dieser Bremsstift 176 aus der Bohrung 174 hervor. An diesem Ende des Stiftes 176 ist ein Bremsklotz 178 angebracht. An dem anderen Ende des Bremsstiftes 176 ist ein zweiter Dauermagnet 180 befestigt, der an der dem ersten Dauermagneten 158 gegenüberliegenden Seite der Ringspule 166 angeordnet ist. Die Polarisierung des Dauermagneten 180 ist derart, daß die zueinander nächstliegenden Seiten der beiden Dauermagnete 158 und 180 gleiche Pole haben. In der FIG 3 ist der Nordpol des Dauermagneten 180, bezeichnet mit N, links angegeben, während der Südpol, bezeichnet mit S, rechts angegeben ist.

Wird nun die Ringspule 166 mit einem Strom in der ersten Stromflußrichtung beaufschlagt, dann wird, wie schon beschrieben, der Dauermagnet 158 von der Ringspule 166 weggedrückt. Gleichzeitig wird der Dauermagnet 180 von der Ringspule 166 angezogen wird. In diesem Zustand berührt der Dauermagnet 180 den Spulenkörper 168. Dabei entsteht ein Abstand zwischen dem Bremsklotz 178 und der Verzahnung 58 des Kegelrades 56, d.h. die Bremse 172 ist nicht in Eingriff, das Kegelrad 56 ist über die Antriebsachse 22 frei drehbar. Wird die Stromflußrichtung in der Spule 166 umgedreht, dann wird der Dauermagnet 158, wie schon beschrieben, von der Spule 166 angezogen, während der Dauermagnet 180 von der Spule 166 abgestoßen wird, d.h. die Kupplung 140 wird gelöst, und die Bremse 172 wirkt mit ihrem Bremsklotz 178 auf die Verzahnung 58 des Kegelrades 56. Damit wird die Stellung, in der sich der Ultraschall-Wandler 24 gerade befindet, fixiert. Von der Antriebsachse 22 kann jetzt nur noch der Ultraschall-Wandler 90 angetrieben werden. Das Kegelrad 56 steht fest, während sich das Halteteil 61 um den Zapfen 146 des Kegelrades 56 dreht.

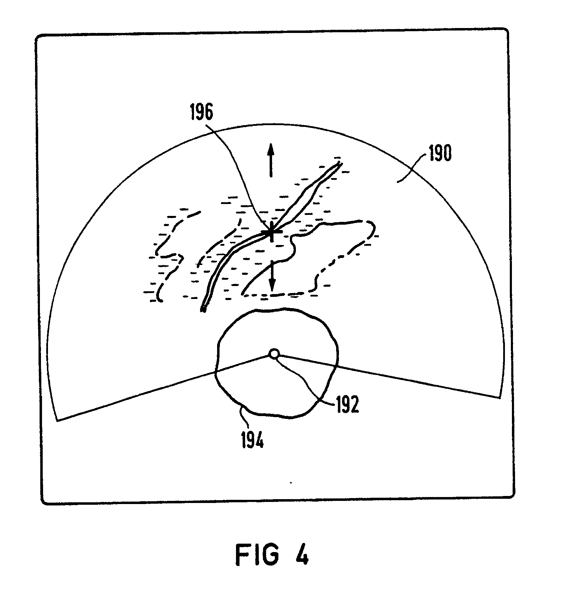

In FIG 4 ist eine Abbildung, z.B. auf dem Monitor der Ultraschall-Abtasteinrichtung, der vom Ultraschall-Wandler 90 abgetasteten Untersuchungsfläche gezeigt. Es handelt sich um eine kreissektorförmige Fläche in der Ebene, die in FIG 3 mit A-A bezeichnet ist. Der Sektorursprung 192 ist die Längsachse der Antriebsachse 22. Eine annähernd kreisförmige Linie 194, die sich um den Sektorursprung 192 befindet, stellt die Wandung der Körperhöhle dar, in der sich der Untersuchungskopf 4 befindet. Zur Einleitung der Doppler-Flußmessung wird der gesamte Untersuchungskopf 4 der Ultraschall-Abtasteinrichtung 2 in der Körperhöhle solange gedreht, bis sich das zu untersuchende Gefäß oder auch "region of interest" (ROI) in der Mitte des Sektorbildes 190 befindet. Nun wird eine auf dem Monitorbild A-A verschiebbare Marke 196 auf die zu untersuchende Stelle des Gefäßes (ROI) geschoben. Die Stellung der Marke 196 auf dem Monitorbild bestimmt den Winkel alpha, den der Ultraschall-Wandler 24 bezüglich der Längsachse der Antriebswelle 22 einnehmen muß. Dieser Winkel wird in der Ultraschall-Abtasteinrichtung 2 aus der Stellung der Marke 196 berechnet. Auf diesen berechneten Winkel alpha wird nun der Ultraschall-Wandler 24 im Langsamgang eingestellt. Deckt sich der Sollwert mit dem Istwert des Winkels alpha, wird die Kupplung 140 gelöst, und dabei gleichzeitig die Bremse 172 betätigt. Der Ultraschall-Wandler 24 wird dann in der Ultraschall-Abtasteinrichtung 2 auf Doppler-Flußmessung umgeschaltet, d.h. er mißt die Geschwindigkeit der Flüssigkeit im Gefäß über die Frequenzverschiebung des ausgesandten Ultraschallimpulses. Während der Doppler-Flußmessung kann gleichzeitig der Ultraschall-Wandler 90 in der Ebene A-A ein B-Sektor-Scan durchführen, d.h. der Ort der Doppler-Flußmessung ist im Sektorbild A-A auf dem Monitor genau beobachtbar. Eine Lageveränderung des Untersuchungskopfes 4 oder des Gefäßes im Körper wird sofort erkannt.

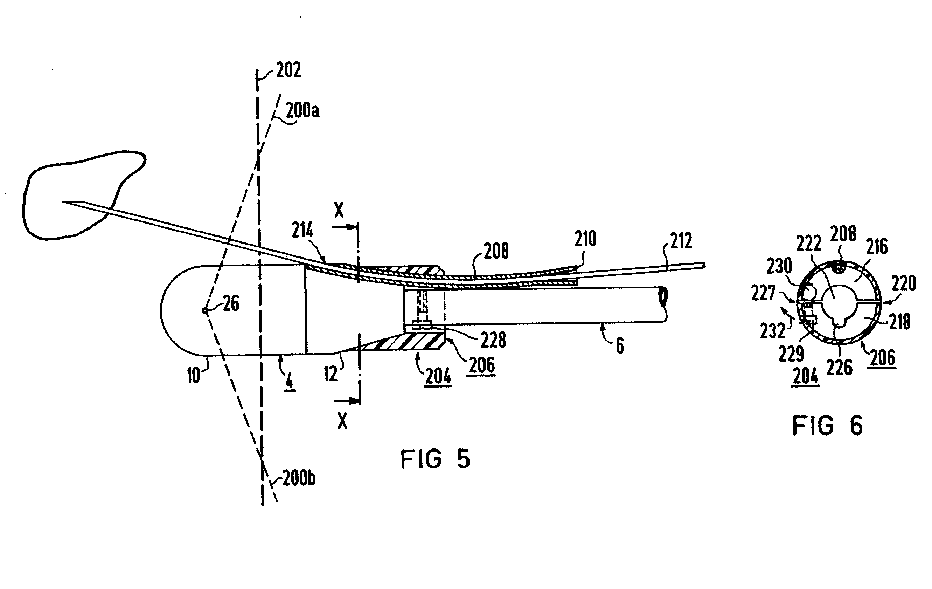

In FIG 5 ist wiederum mit 4 der Untersuchungskopf einer Ultraschall-Abtasteinrichtung 2 bezeichnet. Die beiden gestrichelten Linien 200a, 200b, die ihren Ursprung in der Achse 26 haben, geben die Grenze der Schnitt- oder Untersuchungsebene, die vom Ultraschall-Wandler 24 abgetastet wird. Diese Untersuchungsebene liegt hier in der Papierebene. Die zweite Schnitt- oder Untersuchungsebene, die durch den Ultraschall-Wandler 90 abgetastet wird, der hier -wie in FIG 3 - senkrecht zur Antriebsachse 22 abtastet, ist durch die gestrichelte Linie 202 angedeutet. Diese Untersuchungsebene steht senkrecht auf der Papierebene. Der Untersuchungskopf 4 ist über die Führung 6 mit dem hier nicht dargestellten Gehäusekopf 8 verbunden. Die Klemmutter 12 hält auch hier, wie in FIG 1 erläutert, die Umhüllung 10 am Untersuchungskopf 4 fest.

Am Übergang von der Führung 6 zu der Klemmutter 12 ist eine Biopsienadelführung 204 angeordnet. Die Biopsienadelführung 204 besteht aus einer Klemmvorrichtung 206 und aus einem gebogenen Führungsrohr 208. Das Führungsrohr 208 ist in Richtung der Antriebsachse 22 (hier nicht gezeigt), also in der Längsrichtung der Führung 6 angeordnet. Es berührt sowohl die Führung 6 als auch die Klemmutter 12. Das Führungsrohr 208 ist an seinem proximalen Ende oder seiner Einlaßöffnung 210 trichterförmig erweitert, so daß eine Biopsienadel 212 leicht einführbar ist. Außerdem ist die proximale Öffnung 210 schräg zur Längsachse der Antriebsachse 22 ausgerichtet, d.h. die Biopsienadel 212 entfernt sich von der Führung 6 mit zunehmender Entfernung von der Klemmvorrichtung 206. Durch dieses Schrägstellen der proximalen Öffnung 210 wird die Biopsienadel 212 am hier nicht gezeigten Gehäusekopf 8 vorbeigeführt. Dadurch ist die Handhabung der Biopsienadel 212 erleichtert.

Das distale Ende oder die Auslaßöffnung 214 des Führungsrohres 208 ist ebenfalls schräg zur Antriebsachse 22 bzw. zur Führung 6 ausgerichtet. Eine im Führungsrohr 208 geführte Biopsienadel 212 hat somit auch an ihrem distalen Ende eine schräge Ausrichtung bezüglich der Führung 6. Dadurch werden auf einfache Art Gebiete im Körper eines Patienten erreicht, die hinter der Wandung der Körperhöhle liegen. Z.B. ist es auf diese Weise möglich, die Prostata über den Darm zu biopsieren.

Die Biopsienadelführung 204 ist so auf dem Untersuchungskopf 4 befestigt, daß die Biopsienadel während der Untersuchung in der Untersuchungsebene liegt, die von dem ersten Ultraschall-Wandler 24 abgetastet wird. Gleichzeitig ist die Biopsienadel 212 auch in der zweiten Untersuchungsebene zu beobachten, die der Ultraschall-Wandler 90 abtastet. Der Weg der Biopsienadel 212 im Körper ist über die beiden Ultraschall-Untersuchungsebenen genau beobachtbar. Damit sind Möglichkeiten geschaffen, auch unter schwierigen Umständen Untersuchungen durchzuführen. Das Untersuchungsrisiko ist dabei kleingehalten.

Die FIG 6 zeigt die Biopsienadelführung 204 in einer Ansicht, die in FIG 5 mit X-X bezeichnet ist. Die Klemmvorrichtung 206 ist außen im wesentlichen zylinderförmig. Sie besteht aus zwei Halteteilen 216, 218, die über ein Scharnier 220 miteinander verbunden sind. Dabei ist das Führungsrohr 208 im Halteteil 216 befestigt. Die Klemmvorrichtung 206 weist im geschlossenen Zustand eine zentrale Öffnung 222 auf. Die Kontur der zentralen Öffnung 222 ist wenigstens teilweise der Außenkontur des Untersuchungskopfes 4 an der Übergangsstelle zur Führung 6 angepaßt. Hier hat die Öffnung 222 einen zylindrischen und einen konischen Teil. Die zwei Halteteile 216, 218 haben die Form eines Halbzylinders, die bei geschlossener Klemmvorrichtung 206 einen Zylinder bilden, mit einem wichtigen Unterschied, daß sie sich bei geschlossener Klemmvorrichtung 206 nicht berühren.

Beide Halteteile 216, 218 bestehen bevorzugt aus einem elastischen Kunststoff, z.B. aus Polypropylen.

Das Scharnier 220, das die zwei Halteteile 216, 218 miteinander verbindet, ist hier ein sogenanntes Filmscharnier 220. Dabei werden beide Halteteile 216, 218 und das Filmscharnier 220 vorteilhafterweise gemeinsam im Spritzgießverfahren hergestellt.

In dem zylindrischen Teil der Öffnung 222 ist eine Nut 226 eingebracht. Diese Nut nimmt eine Rippe 228 auf, die sich auf der Führung 6 befindet und die in FIG 5 gezeigt ist. Dadurch ist die relative Lage der Biopsienadelführung 204 zum Untersuchungskopf 4 festgelegt.

Ein Verschluß 227 für die Klemmvorrichtung 206 ist an der dem Scharnier 220 gegenüberliegenden Seite angeordnet. Der Verschluß 227 besteht aus einer Schraube 229, die sich in einen Bolzen 230 einschrauben läßt. Dieser Bolzen 230 ist in dem Halteteil 216 drehbar gelagert. Ein im Halteteil 218 eingebrachter Schlitz erlaubt, die Schraube 229 in Richtung des Pfeiles 232 wegzuschwenken. Dann läßt sich die Klemmvorrichtung auseinanderklappen.

Die gesamte Biopsienadelführung 204 besteht aus heißdampfsterilisierbarem Material. Vor der Anwendung zur Biopsie wird über den Untersuchungskopf 4 ein sterilisierter dünner Überzug geschoben. Danach wird die sterilisierte Biopsienadelführung 204 in aufgeklapptem Zustand so an den Untersuchungskopf 4 gehalten, daß die Rippe 228 in der Nut 226 liegt. Die Nut 226 und die Rippe 228 bilden eine Führung, die die relative Lage der Biopsienadelführung 204 zum Untersuchungskopf 4 festlegt. Die Klemmvorrichtung 206 wird mit dem Verschluß 227 geschlossen, d.h. die beiden Halteteile 216, 218 werden über die Schraube 229 festgehalten. Damit ist die Biopsienadelführung 204 in definierter Lage auf dem Untersuchungskopf 4 befestigt.

Dadurch, daß die äußeren Abmessungen der Biopsienadelführung 204 nicht über die äußeren Abmessungen des Untersuchungskopfes 4 hinausragen, ist das Einführen des Untersuchungskopfes 4 in eine Körperhöhle nicht behindert. Der Untersuchungskopf 4 wird in der Körperhöhle so ausgerichtet, daß das zu untersuchende Gebiet (ROI) in der Untersuchungsebene liegt, die der Ultraschall-Wandler 24 abtastet. Durch die feste, definierte Lage des Führungsrohres 208 in Bezug auf den Untersuchungskopf 4 ist sichergestellt, daß die Biopsienadel 212 nur in dieser Ebene eingebracht werden kann. Bei der gleichzeitig möglichen Beobachtung des Zuführweges in der weiteren Untersuchungsebene, die vom Ultraschallkopf 90 abgetastet wird, können evtl. vorhandene Hindernisse, die in der ersten Untersuchungsebene nicht sichtbar sind, erkannt werden.

## Ansprüche

1. Intrakavitäre Ultraschall-Abtasteinrichtung mit einem Untersuchungskopf, in dessen Innenraum ein Ultraschall-Wandler untergebracht ist, der um eine Achse (26) drehbar und dabei mittels einer Antriebsachse (22) antreibbar ist, wobei die genannte Achse (26) im wesentlichen senkrecht zum distalen Ende der Antriebsachse (22) ausgerichtet ist, und zum Drehen des Ultraschall-Wandlers (24) um die Achse (26) ein erstes Zahnrad (52) vorgesehen ist, das mit einem zweiten, am distalen Ende der Antriebsachse (22) befestigten Zahnrad (56) im Eingriff steht, **dadurch gekennzeichnet, daß** ein mit der Antriebsachse (22) verbundenes Halteteil (61) vorgesehen ist, an dem ein weiterer Ultraschall-Wandler (90) befestigt ist.

2. Ultraschall-Abtasteinrichtung nach Anspruch 1, **dadurch gekennzeichnet, daß** das erste und zweite Zahnrad als ein erstes bzw. zweites Kegelrad (52, 56) ausgebildet sind, und daß das zweite Kegelrad (56) eine distal gelegene Mulde (60) aufweist, in die der Ultraschall-Wandler (24) beim Drehen um die Achse (26) eintaucht.

3. Ultraschall-Abtasteinrichtung nach Anspruch 1 oder 2, **dadurch gekennzeichnet,** daß der Ultraschall-Wandler (24) an einer Trommel (30) befestigt ist, die um eine entlang der Achse (26) ausgerichtete Lagerachse (42) drehbar ist, daß die Trommel (30) an ihrer einen Stirnseite das erste Zahnrad (52) trägt, dessen Zähne (54) mit den Zähnen (58) des zweiten Zahnrades (56) im Eingriff stehen, und daß das zweite Zahnrad (56) zentrisch auf dem distalen Ende der Antriebsachse (22) befestigt ist.

4. Ultraschall-Abtasteinrichtung nach Anspruch 3, **dadurch gekennzeichnet,** daß die Lagerachse (42) als Hohlachse ausgebildet ist.

5. Ultraschall-Abtasteinrichtung nach Anspruch 3 oder 4, **dadurch gekennzeichnet,** daß die Trommel (30) an ihrer anderen Stirnseite mit dem drehbaren Teil (72, 76) eines Drehtransformators (70) versehen ist, und daß der nicht-drehbare Teil (78, 84) des Drehtransformators (70) mit der Lagerachse (42) verbunden ist.

6. Ultraschall-Abtasteinrichtung nach Anspruch 3, 4 oder 5, **dadurch gekennzeichnet,** daß die Lagerachse (42) an einem Gehäuse (48) befestigt ist, das die beiden Zahnräder (52, 56) umschließt und mit einem Ultraschall-Fenster (50) für den Durchgang der vom Ultraschall-Wandler (24) abgestrahlten Ultraschall-Impulse und der von diesem aufgenommenen Echos versehen ist.

7. Ultraschall-Abtasteinrichtung nach Anspruch 6, **dadurch gekennzeichnet,** daß das Gehäuse (48) von einer elastischen Umhüllung (10) umgeben ist.

8. Ultraschall-Abtasteinrichtung nach einem der Ansprüche 3 bis 7, **dadurch gekennzeichnet,** daß die Trommel (30) mittels mindestens eines Kugellagers (38, 40) auf der Lagerachse (42) drehbar angeordnet ist.

9. Ultraschall-Abtasteinrichtung nach einem der Ansprüche 1 bis 8, **dadurch gekennzeichnet,** daß die Abstrahlrichtung des weiteren Ultraschall-Wandlers (90) schräg zur Radialebene der Antriebsachse (22) ausgerichtet ist.

10. Ultraschall-Abtasteinrichtung nach einem der Ansprüche 1 bis 9, **dadurch gekennzeichnet,** daß die Antriebsachse (22) als Hohlachse ausgebildet ist.

11. Ultraschall-Abtasteinrichtung nach einem der Ansrprüche 1 bis 10, **dadurch gekennzeichnet,** daß die Antriebsachse (22) in einem Kugellager (64) läuft, das vom Gehäuse (48) gehalten ist.

12. Ultraschall-Abtasteinrichtung nach einem der Ansprüche 1 bis 11, bei der der Untersuchungskopf über ein Führung an einem Gehäusekopf befestigt ist, **dadurch gekennzeichnet,** daß die Antriebsachse (22) im Gehäusekopf (8) an der Antriebswelle (104) eines elektrischen Motors (106) befestigt ist.

13. Ultraschall-Abtasteinrichtung nach Anspruch 12, **dadurch gekennzeichnet,** daß am proximalen Ende der Antriebswelle (104) das drehbare Teil (126, 128) eines Drehtransformators (124) angeordnet ist.

14. Ultraschall-Abtasteinrichtung nach Anspruch 12 oder 13, **dadurch gekennzeichnet,** daß die Antriebsachse (22) als Hohlachse ausgebildet ist, die am proximalen Ende (22a, 22b) eine größere Wandstärke und/oder einen größeren Durchmesser besitzt als am distalen Ende.

15. Ultraschall-Abtasteinrichtung nach einem der Ansprüche 13 und 14, **dadurch gekennzeichnet,** daß das drehbare Teil (126, 128) des Drehtransformators (124) an der proximalen Stirnseite der Antriebsachse (22) befestigt ist.

16. Ultraschall-Abtasteinrichtung nach einem der Ansprüche 12 bis 15, **dadurch gekennzeichnet,** daß die Antriebsachse (22) zwischen dem Untersuchungskopf (4) und dem Gehäusekopf (8) von einem Trägerrohr (94) umgeben ist, das mit einer Kunststoff-Umhüllung (14) versehen ist.

17. Ultraschall-Abtasteinrichtung nach einem der Ansprüche 12 bis 16, **dadurch gekennzeichnet,** daß im Gehäusekopf (8) eine Handpumpe (16) angeordnet ist, die zum Aufpumpen einer den Untersuchungskopf (4) umgebenden Umhüllung (10) mit Ultraschall-Ankoppelflüssigkeit (66) dient.

18. Ultraschall-Abtasteinrichtung nach Anspruch 16 und 17, **dadurch gekennzeichnet,** daß die Handpumpe (16) und der Untersuchungskopf (4) durch eine hydraulische Leitung (133) miteinander verbunden sind.

19. Ultraschall-Abtasteinrichtung nach Anspruch 18, **dadurch gekennzeichnet,** daß die hydraulische Leitung (133) in einen Hohlraum (92) in der Führung (6) mündet.

20. Ultraschall-Abtasteinrichtung nach einem der Ansprüche 1 bis 19, **dadurch gekennzeichnet,** daß der erste Ultraschall-Wandler (90) fest mit der Antriebsachse (22) gekoppelt ist und daß der zweite Ultraschall-Wandler (24) über eine lösbare Kupplung (140) mit der Antriebsachse (22) gekoppelt ist.

21. Ultraschall-Abtasteinrichtung nach Anspruch 20, **dadurch gekennzeichnet,** daß eine Bremse (172) vorgesehen ist, die ein Drehen des zweiten Ultraschall-Wandlers (24) verhindert, wenn er von der Antriebsachse (22) abgekoppelt ist.

22. Ultraschall-Abtasteinrichtung nach Anspruch 20 oder 21, **dadurch gekennzeichnet,** daß die Kupplung (140) das zweite Zahnrad (56) mit der Antriebsachse (22) mechanisch verbindet.

23. Ultraschall-Abtasteinrichtung nach einem der Ansprüche 20 bis 22, **dadurch gekennzeichnet,** daß der zweite Ultraschall-Wandler (24), wenn er mit der Antriebsachse (22) gekoppelt ist, zur Erzeugung des B-Bildes vorgesehen ist, und wenn er von der Antriebsachse (22) abgekoppelt ist, zur Durchführung einer Doppler-Flußmessung vorgesehen ist.

24. Ultraschall-Abtasteinrichtung nach Anspruch 23, **dadurch gekennzeichnet,** daß der Ort der Doppler-Flußmessung im B-Bild darstellbar ist.

25. Ultraschall-Abtasteinrichtung nach einem der Ansprüche 22 bis 24, **dadurch gekennzeichnet,** daß die Kupplung (140) einen Stift (154) umfaßt, der in einer zur Antriebsachse (22) parallelen Bohrung (152) im Halteteil (61) verschiebbar ist, und daß im zweiten Zahnrad (56) eine Aussparung (160) eingebracht ist, in die ein Ende (162) des Stiftes (154) eingreift, wenn der zweite Ultraschall-Wandler (24) mit der Antriebsachse (22) gekoppelt ist.

26. Ultraschall-Abtasteinrichtung nach Anspruch 25, **dadurch gekennzeichnet,** daß am anderen Ende (156) des Stiftes (154) eine erster Dauermagnet (158) befestigt ist und daß konzentrisch um die Antriebsachse (22) eine Ringspule (166) angeordnet ist, die in magnetische Wechselwirkung mit dem ersten Dauermagneten (158) derart treten kann, daß bei einer ersten Stromflußrichtung der Stift (154) in die Aussparung (160) hineingedrückt wird und daß bei der zweiten Stromflußrichtung der Stift (154) aus der Aussparung (160) herausgezogen wird.

27. Ultraschall-Abtasteinrichtung nach Anspruch 26, **dadurch gekennzeichnet,** daß die Bremse (172) einen Bremsstift (176) umfaßt, der in einer zur Antriebsachse (22) parallelen Bohrung (174) im Gehäuse (48) verschiebbar ist, daß an einem Ende des Bremsstiftes (176) ein Bremsklotz (178) befestigt ist und daß an dem anderen Ende des Bremstiftes (174) ein zweiter Dauermagnet (180) befestigt ist, der auf der Seite der Ringspule (166) angeordnet ist, die dem ersten Dauermagneten (158) gegenüberliegt, wobei die Ringspule (166 ) derart in magnetische Wechselwirkung mit dem zweiten Dauermagneten treten kann, daß bei der ersten Stromflußrichtung der Bremsklotz (178) das zweite Zahnrad (56) nicht berührt und daß bei der zweiten Stromflußrichtung der Bremsklotz (178) gegen das zweite Zahnrad (56 ) drückt.

28. Ultraschall-Abtasteinrichtung nach einem der Ansprüche 20 bis 27, **dadurch gekennzeichnet,** daß das zweite Zahnrad (56) einen zentralen Zapfen (146) aufweist, der in einer ebenfalls zentralen Bohrung (144) im Halteteil (61) gelagert ist.

29. Ultraschall-Abtasteinrichtung nach einem der Ansprüche 1 bis 28, **dadurch gekennzeichnet,** daß eine Biopsienadelführung (204), die ein Fürungsrohr (208) und eine Klemmvorrichtung (206) umfaßt, mit dem Untersuchungskopf (4) lösbar verbunden ist, daß das Führungsrohr (208) seitlich versetzt und in Richtung der Antriebsachse (22) angeordnet ist und daß die Auslaßöffnung (214) des Führungsrohrs (208) schräg zur Antriebsachse (22) ausgerichtet ist.

30. Ultraschall-Abtasteinrichtung nach Anspruch 29, **dadurch gekennzeichnet,** daß die Klemmvorrichtung (206) zwei Halteteile (216, 218) umfaßt, die im geschlossenen Zustand eine zentrale Öffnung (222) bilden, deren Kontur der Außenkontur des Untersuchungskopfes (4) wenigstens teilweise angepaßt ist und daß die zwei Halteteile (216, 218) über ein Scharnier (220) miteinander verbunden sind.

31. Ultraschall-Abtasteinrichtung nach Anspruch 29 oder 30, **dadurch gekennzeichnet,** daß die Einlaßöffnung (210) des Führungsrohres (218) ebenfalls schräg zur Antriebsachse (22) ausgerichtet ist.

32. Ultraschall-Abtasteinrichtung nach einem der Ansprüche 29 bis 31, **dadurch gekennzeichnet,** daß die Einlaßöffnung (210) des Führungsrohres (208) trichterförmig ausgebildet ist.

33. Ultraschall-Abtasteinrichtung nach einem der Ansprüche 29 bis 32, **dadurch gekennzeichnet,**
daß eine Führung (226, 228) vorgesehen ist, die die relative Lage der Biopsienadelführung (204) zum Untersuchungskopf (4) so festlegt, daß eine im Führungsrohr (208) geführte Biopsienadel (212) in einer der Schnittebenen des Untersuchungskopfes (4) liegt.

34. Ultraschall-Abtasteinrichtung nach einem der Ansprüche 29 bis 22, **dadurch gekennzeichnet,**
daß die äußeren Abmessungen der Biopsienadelführung (204) nicht über die äußeren Abmessungen des Untersuchungskopfes (4) hinausragen.

## Claims

1. Intracavitary ultrasonic scanning apparatus with an examination head, in the inner space of which there is accommodated an ultrasonic transducer, which can be rotated around an axis (26) and in this connection can be driven by means of a drive axis (22), whereby the named axis (26) is aligned substantially perpendicularly to the distal end of the drive axis (22), and for the rotation of the ultrasonic transducer (24) around the axis (26) there is provided a first gear wheel (52), which is engaged with a second gear wheel (56) fastened to the distal end of the drive axis (22), characterized in that there is provided a holding part (61) connected to the drive axis (22), to which holding part there is fastened a further ultrasonic transducer (90).

2. Ultrasonic scanning apparatus according to claim 1, characterized in that the first and second gear wheel are constructed as a first or second bevel-gear wheel (52, 56), and in that the second bevel-gear wheel (56) has a distally situated depression (60), into which the ultrasound transducer (24) dips with rotation around the axis (26).

3. Ultrasonic scanning apparatus according to claim 1 or 2, characterized in that the ultrasonic transducer (24) is fastened to a drum (30), which is rotatable around a bearing axis (42) aligned along the axis (26), in that the drum (30) at one of its front sides carries the first gear wheel (52), the teeth (54) of which are engaged with the teeth (58) of the second gear wheel (56), and in that the second gear wheel (56) is centrally fastened on the distal end of the drive axis (22).

4. Ultrasonic scanning apparatus according to claim 3, characterized in that the bearing axis (42) is constructed as hollow axis.

5. Ultrasonic scanning apparatus according to claim 3 or 4, characterized in that the drum (30) at its other front side is provided with the rotatable part (72, 76) of a rotary transformer (70), and in that the non-rotatable part (78, 84) of the rotary transformer (70) is connected to the bearing axis (42).

6. Ultrasonic scanning apparatus according to claim 3, 4 or 5, characterized in that the bearing axis (42) is fastened to a housing (48), which encloses the two gear wheels (52, 56) and is provided with an ultrasonic window (50) for the passage of the ultrasonic pulses, emitted by the ultrasonic transformer (24), and the echoes received by this.

7. Ultrasonic scanning apparatus according to claim 6, characterized in that the housing (48) is surrounded by an elastic casing (10).

8. Ultrasonic scanning apparatus according to one of claims 3 to 7, characterized in that the drum (30) is rotatably arranged on the bearing axis (42) by means of at least one ball bearing (38, 40).

9. Ultrasonic scanning apparatus according to one of claims 1 to 8, characterized in that the beam direction of the further ultrasonic transducer (90) is aligned at inclined orientation to the radial plane of the drive axis (22).

10. Ultrasonic scanning apparatus according to one of claims 1 to 9, characterized in that the drive axis (22) is constructed as hollow axis.

11. Ultrasonic scanning apparatus according to one of claims 1 to 10, characterized in that the drive axis (22) runs in a ball bearing (64) which is held by the housing (48).

12. Ultrasonic scanning apparatus according to one of claims 1 to 11, in which the examination head is fastened to a housing head by means of a guide, characterized in that the drive axis (22) in the housing head (8) is fastened to the drive shaft (104) of an electrical motor (106).

13. Ultrasonic scanning apparatus according to claim 12, characterized in that the rotatable part (126, 128) of a rotary transformer (124) is arranged on the proximal end of the drive shaft (104).

14. Ultrasonic scanning apparatus according to claim 12 or 13, characterized in that the drive axis (22) is constructed as hollow axis, which on the proximal end (22a, 22b) has a greater wall thickness and/or a greater diameter than on the distal end.

15. Ultrasonic scanning apparatus according to one of claims 13 and 14, characterized in that the rotatable part (126, 128) of the rotary transformer (124) is fastened to the proximal front side of the drive axis (22).

16. Ultrasonic scanning apparatus according to one of claims 12 to 15, characterized in that the drive axis (22) between the examination head (4) and the housing head (8) is surrounded by a carrier tube (94) which is provided with a synthetic-material casing (14).

17. Ultrasonic scanning apparatus according to one of claims 12 to 16, characterized in that there is arranged in the housing head (8) a hand pump (16) which serves to pump up a casing (10) surrounding the examination head (4) with ultrasonic coupling fluid (66).

18. Ultrasonic scanning apparatus according to claim 16 and 17, characterized in that the hand pump (16) and the examination head (4) are connected to one another by a hydraulic line (133).

19. Ultrasonic scanning apparatus according to claim 18, characterized in that the hydraulic line (133) opens into a cavity (92) in the guide (6).

20. Ultrasonic scanning apparatus according to one of claims 1 to 19, characterized in that the first ultrasonic transformer (90) is firmly coupled to the drive axis (22) and in that the second ultrasonic transformer (24) is coupled by means of a releasable coupling (140) to the drive axis (22).

21. Ultrasonic scanning apparatus according to claim 20, characterized in that a brake (172) is provided, which prevents a rotation of the second ultrasonic transducer (24) if it is decoupled from the drive axis (22).

22. Ultrasonic scanning apparatus according to claim 20 or 21, characterized in that the coupling (140) mechanically connects the second gear wheel (56) to the drive axis (22).

23. Ultrasonic scanning apparatus according to one of claims 20 to 22, characterized in that the second ultrasonic transducer (24), if it is coupled to the drive axis (22), is provided for the production of the B-image, and if it is decoupled from the drive axis (22), is provided for carrying out a Doppler flow measurement.

24. Ultrasonic scanning apparatus according to claim 23, characterized in that the position of the Doppler flow measurement can be represented in the B-image.

25. Ultrasonic scanning apparatus according to one of claims 22 to 24, characterized in that the coupling (140) comprises a pin (154), which can be displaced in a bore (152) parallel to the drive axis (22) in the holding part (61), and in that there is introduced in the second gear wheel (56) a recess (160), into which an end (162) of the pin (154) engages, if the second ultrasonic transducer (24) is coupled to the drive axis (22).

26. Ultrasonic scanning apparatus according to claim 25, characterized in that a first permanent magnet (158) is fastened to the other end (156) of the pin (154), and in that there is concentrically arranged around the drive axis (22) an annular coil (166), which can enter into magnetic interaction with the first permanent magnet (158) in such a way that in a first current flow direction the pin (154) is pressed into the recess (160) and in that in the second current flow direction the pin (154) is pulled out of the recess (160).

27. Ultrasonic scanning apparatus according to claim 26, characterized in that the brake (172) comprises a brake pin (176) which can be displaced in a bore (174) parallel to the drive axis (22) in the housing (48), in that a brake pad (178) is fastened to one end of the brake pin (176) and in that there is fastened to the other end of the brake pin (174) a second permanent magnet (180), which is arranged on the side of the annular coil (166) which lies opposite the first permanent magnet (158), whereby the annular coil (166) can enter into magnetic interaction with the second permanent magnet in such a way that in the first current flow direction the brake pad (178) does not touch the second gear wheel (56) and in that in the second current flow direction the brake pad (178) presses against the second gear wheel (56).

28. Ultrasonic scanning apparatus according to one of claims 20 to 27, characterized in that the second gear wheel (56) has a central lug (146) which is mounted in a likewise central bore (144) in the holding part (61).

29. Ultrasonic scanning apparatus according to one of claims 1 to 28, characterized in that a biopsy needle guide (204), which comprises a guide tube (208) and a clamping device (206), is connected to the examination head (4) in a releasable manner, in that the guide tube (208) is arranged laterally displaced and in the direction of the drive axis (22) and in that the outlet opening (214) of the guide tube (208) is aligned at inclined orientation to the drive axis (22).

30. Ultrasonic scanning apparatus according to claim 29, characterized in that the clamping device (206) comprises two holding parts (216, 218) which in the closed state form a central opening (222), the contour of which is adapted at least partially to the outer contour of the examination head (4) and in that the two holding parts (216, 218) are connected to one another by means of a hinge (220).

31. Ultrasonic scanning apparatus according to claim 29 or 30, characterized in that the inlet opening (210) of the guide tube (218) is aligned likewise at inclined orientation to the drive axis (22).

32. Ultrasonic scanning apparatus according to one of claims 29 to 31, characterized in that the inlet opening (210) of the guide tube (208) is constructed in the shape of a cone.

33. Ultrasonic scanning apparatus according to one of claims 29 to 32, characterized in that a guide (226, 228) is provided, which sets the position of the biopsy needle guide (204) relative to the examination head (4) in such a way that a biopsy needle (212) guided in the guide tube (208) lies in one of the section planes of the examination head (4).

34. Ultrasonic scanning apparatus according to one of claims 29 to 22 [sic], characterized in that the outer dimensions of the biopsy needle guide (204) do not project over the outer dimensions of the examination head (4).

## Revendications

1. Dispositif intracavitaire d'exploration à ultrasons, comportant une tête d'examen, dans l'espace intérieur de laquelle est logé un transducteur à ultrasons, qui peut tourner autour d'un axe (26) et peut être entraîné au moyen d'un axe d'entraînement (22), et dans lequel ledit axe (26) est sensiblement perpendiculaire à l'extrémité distale de l'axe d'entraînement (22), et pour l'entraînement en rotation du transducteur à ultrasons (24) autour de l'axe (26), il est prévu un premier pignon (52) qui engrène avec un second pignon (56) fixé sur l'extrémité distale de l'axe d'entraînement (22), caractérisé par le fait qu'il est prévu une partie de maintien (61), qui est reliée à l'axe d'entraînement (22) et à laquelle est fixé un autre transducteur à ultrasons (90).

2. Dispositif d'exploration à ultrasons suivant la revendication 1, caractérisé par le fait que les premier et second pignons sont réalisés sous la forme de premier et second pignons coniques (52,56) et que le second pignon conique (56) comporte un évidement distal (60), dans lequel le transducteur à ultrasons (24) pénètre lors de la rotation autour de l'axe (26).

3. Dispositif d'exploration à ultrasons suivant la revendication 1 ou 2, caractérisé par le fait que le transducteur à ultrasons (24) est fixé sur un tambour (30), qui peut tourner autour d'un axe de support (42) qui possède l'orientation de l'axe (26), que le tambour (30) porte, sur l'une de ses faces frontales, le premier pignon (52), dont les dents (54) engrènent avec les dents (58) du second pignon (56), et que le second pignon (56) est fixé en étant centré sur l'extrémité distale de l'axe d'entraînement (22).

4. Dispositif d'exploration à ultrasons suivant la revendication 3, caractérisé par le fait que l'axe de support (42) est réalisé sous la forme d'un axe creux.

5. Dispositif d'exploration à ultrasons suivant la revendication 3 ou 4, caractérisé par le fait que le tambour (30) comporte, sur son autre face frontale, la partie rotative (72,76) d'un transformateur rotatif (70), et que la partie non rotative (78,84) du transformateur rotatif (70) est reliée à l'axe de support (42).

6. Dispositif d'exploration à ultrasons suivant les revendications 3,4 ou 5, caractérisé par le fait que l'axe de support (42) est fixé sur un boîtier (48), qui entoure les deux pignons (52,56) et comporte une fenêtre (50) de passage des ultrasons, qui permet le passage des impulsions ultrasonores émises par le transducteur à ultrasons (24) et des échos reçus par ce transducteur.

7. Dispositif d'exploration à ultrasons suivant la revendication 6, caractérisé par le fait que le boîtier (48) est entouré par une enveloppe élastique (10).

8. Dispositif d'exploration à ultrasons suivant l'une des revendications 3 à 7, caractérisé par le fait que le tambour (30) est monté sur l'axe de support (42) de manière à pouvoir tourner, à l'aide d'au moins un roulements à billes (38,40).

9. Dispositif d'exploration à ultrasons suivant l'une des revendications 1 à 8, caractérisé par le fait que la direction d'émission de l'autre transducteur à ultrasons (90) est oblique par rapport au plan radial de l'axe d'entraînement (22).

10. Dispositif d'exploration à ultrasons suivant l'une des revendications 1 à 9, caractérisé par le fait que l'axe d'entraînement (22) est réalisé sous la forme d'un axe creux.

11. Dispositif d'exploration à ultrasons suivant l'une des revendications 1 à 10, caractérisé par le fait que l'axe d'entraînement (22) passe à l'intérieur d'un roulement à billes (64), qui est maintenu par le boîtier (48).

12. Dispositif d'exploration à ultrasons suivant l'une des revendications 1 à 11, dans lequel la tête d'examen est fixée au moyen d'un guide sur une tête du boîtier, caractérisé par le fait que l'axe d'entraînement (22) est fixé dans la tête (8) du boîtier, sur l'arbre d'entraînement (104) d'un moteur électrique (106).

13. Dispositif d'exploration à ultrasons suivant la revendication 12, caractérisé par le fait que la partie rotative (126,128) d'un transformateur rotatif (124) est installée sur l'extrémité proximale de l'arbre d'entraînement (104).

14. Dispositif d'exploration à ultrasons suivant la revendication 12 ou 13, caractérisé par le fait que l'axe d'entraînement (22) est réalisé sous la forme d'un axe creux, qui possède, sur son extrémité proximale (22a,22b), une épaisseur de paroi plus importante et/ou un diamètre plus grand qu'au niveau de l'extrémité distale.

15. Dispositif d'exploration à ultrasons suivant l'une des revendications 13 et 14, caractérisé par le fait que la partie rotative (126,128) du transformateur rotatif (124) est fixée sur la face frontale proximale de l'axe d'entraînement (22).

16. Dispositif d'exploration à ultrasons suivant l'une des revendications 12 à 15, caractérisé par le fait que l'axe d'entraînement (22) est entouré, entre la tête d'examen (4) et la tête (8) du boîtier, par un tube de support (94) comportant une enveloppe en matière plastique (14).

17. Dispositif d'exploration à ultrasons suivant l'une des revendications 12 à 16, caractérisé par le fait que dans la tête (8) du boîtier est disposée une pompe manuelle (16), qui sert à introduire par pompage un liquide (66) de couplage des ultrasons, dans une enveloppe (10) entourant la tête d'examen (4).

18. Dispositif d'exploration à ultrasons suivant les revendications 16 et 17, caractérisé par le fait que la pompe manuelle (16) et la tête d'examen (4) sont reliées entre elles par une canalisation hydraulique (133).

19. Dispositif d'exploration à ultrasons suivant la revendication 18, caractérisé par le fait que la canalisation hydraulique (133) débouche dans une cavité (92) ménagée dans le guide (6).

20. Dispositif d'exploration à ultrasons suivant l'une des revendications 1 à 19, caractérisé par le fait que le premier transducteur à ultrasons (90) est accouplé rigidement à l'axe d'entraînement (22) et que le second transducteur à ultrasons (24) est accouplé à l'axe d'entraînement (22) au moyen d'un accouplement débrayable (140).

21. Dispositif d'exploration à ultrasons suivant la revendication 20, caractérisé par le fait qu'il est prévu un frein (172) empêchant une rotation du second transducteur à ultrasons (24), lorsqu'il est désaccouplé de l'arbre d'entraînement (22).

22. Dispositif d'exploration à ultrasons suivant la revendication 20 ou 21, caractérisé par le fait que l'accouplement (140) relie mécaniquement le second pignon (56) à l'axe d'entraînement (22).

23. Dispositif d'exploration à ultrasons suivant l'une des revendications 20 à 22, caractérisé par le fait que lorsque le second transducteur à ultrasons (24) est accouplé à l'axe d'entraînement (22), il sert à produire l'image B et que, lorsqu'il est désaccouplé de l'axe d'entraînement (22), il sert à exécuter une mesure Doppler de flux.

24. Dispositif d'exploration à ultrasons suivant la revendication 23, caractérisé par le fait que l'emplacement de la mesure Doppler de flux peut être représenté dans l'image B.

25. Dispositif d'exploration à ultrasons suivant l'une des revendications 22 à 24, caractérisé par le fait que l'accouplement (140) comporte une tige (154) qui peut être déplacée dans un perçage (152) parallèle à l'axe d'entraînement (22) et ménagé dans la partie de maintien (61), et que dans le second pignon (56) est ménagé un évidement (160), dans lequel s'engage une extrémité (162) de la tige (154) lorsque le second transducteur à ultrasons (24) est accouplé à l'axe d'entraînement (22).

26. Dispositif d'exploration à ultrasons suivant la revendication 25, caractérisé par le fait qu'un premier aimant permanent (158) est fixé à l'autre extrémité (156) de la tige (154), et qu'autour de l'axe d'entraînement (22) est disposée concentriquement une bobine annulaire (166), qui peut coopérer magnétiquement avec le premier aimant permanent (158) de telle sorte que, dans le cas d'un premier sens du flux de courant, la tige (154) est enfoncée dans l'évidement (160) et que, dans le cas du second sens du flux de courant, la tige (154) est ressortie hors de l'évidement (160).

27. Dispositif d'exploration à ultrasons suivant la revendication 26, caractérisé par le fait que le frein (172) comporte une tige de frein (176), qui est déplaçable dans un perçage (174) parallèle à l'axe d'entraînement (22) et ménagé dans le boîtier (48), qu'un sabot de frein (178) est fixé sur une extrémité de la tige de frein (176) et que sur l'autre extrémité de la tige de frein (174) est fixé un second aimant permanent (180), qui est disposé sur le côté de la bobine annulaire (166), qui est situé en vis-à-vis du premier aimant permanent (158), la bobine annulaire (166) pouvant coopérer magnétiquement avec le second aimant permanent de telle sorte que, dans le cas du premier sens du flux de courant, le sabot de frein (178) n'est pas en contact avec le second pignon (56) et que dans le cas du second sens du flux de courant, le sabot de frein (178) est en appui contre le second pignon (56).

28. Dispositif d'exploration à ultrasons suivant l'une des revendications 20 à 27, caractérisé par le fait que le second pignon (56) possède une broche centrale (146), qui est également logée dans un perçage (144), également central, ménagé dans la partie de retenue (61).

29. Dispositif d'exploration à ultrasons suivant l'une des revendications 1 à 28, caractérisé par le fait qu'un guide (204) pour une aiguille à biopsie (204), qui comporte un tube de guidage (208) et un dispositif de serrage (206), est relié de façon amovible à la tête d'examen (4), que le tube de guidage (208) est décalé latéralement et est disposé dans la direction de l'axe d'entraînement (22), et que l'ouverture de sortie (214) du tube de guidage (208) est oblique par rapport à l'axe d'entraînement (22).

30. Dispositif d'exploration à ultrasons suivant la revendication 29, caractérisé par le fait que le dispositif de serrage (206) comporte deux parties de retenue (226,218), qui forment, à l'état fermé, une ouverture centrale (222), dont le contour est adapté au moins partiellement au contour extérieur de la tête d'examen (4), et que les deux parties de retenue (216,218) sont reliées entre elles par une charnière (220).

31. Dispositif d'exploration à ultrasons suivant la revendication 29 ou 30, caractérisé par le fait que l'ouverture d'admission (210) du tube de guidage (218) est également orientée obliquement par rapport à l'axe d'entraînement (22).

32. Dispositif d'exploration à ultrasons suivant l'une des revendications 29 à 31, caractérisé par le fait que l'ouverture d'admission (210) du tube de guidage (208) est réalisée en forme d'entonnoir.

33. Dispositif d'exploration à ultrasons suivant l'une des revendications 29 à 32, caractérisé par le fait qu'il est prévu un guide (226,228), qui fixe la position relative du guide (204) de l'aiguille à biopsie par rapport à la tête d'examen (4) de telle sorte qu'une aiguille à biopsie (212) guidée dans le tube de guidage (208) est située dans l'un des plans de coupe de la tête d'examen (4).

34. Dispositif d'exploration à ultrasons suivant l'une des revendications 29 à 22, caractérisé par le fait que les dimensions extérieures du guide (204) de l'aiguille à biopsie ne sont pas supérieures aux dimensions extérieures de la tête d'examen (4).

FIG 1

FIG 2

EP 0 273 180 B1

FIG 3

EP 0 273 180 B1

FIG 4

FIG 5

FIG 6